(19) Europäisches Patentamt
European Patent Office
Office européen des brevets

(11) EP 4 273 134 B1

(12) **EUROPEAN PATENT SPECIFICATION**

(45) Date of publication and mention of the grant of the patent:
**29.07.2026 Bulletin 2026/31**

(21) Application number: **22820557.1**

(22) Date of filing: **08.06.2022**

(51) International Patent Classification (IPC):
***C07D 319/12*** *(2006.01)* ***C07B 63/00*** *(2006.01)*

(52) Cooperative Patent Classification (CPC):
**C07D 319/12; C07B 63/00**

(86) International application number:
**PCT/KR2022/008085**

(87) International publication number:
**WO 2022/260430 (15.12.2022 Gazette 2022/50)**

(54) **ISOLATION METHOD FOR LACTIDE RACEMATE**

VERFAHREN ZUR ISOLIERUNG VON LACTIDRACEMAT

PROCÉDÉ D'ISOLEMENT D'UN MÉLANGE RACÉMIQUE DE LACTIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.06.2021 KR 20210075600**
**07.06.2022 KR 20220069132**

(43) Date of publication of application:
**08.11.2023 Bulletin 2023/45**

(73) Proprietor: **LG Chem, Ltd.**
**Seoul 07336 (KR)**

(72) Inventors:
- **AN, Yujin**
  **Daejeon 34122 (KR)**
- **JUNG, Hyunchul**
  **Daejeon 34122 (KR)**
- **OH, Wan Kyu**
  **Daejeon 34122 (KR)**

(74) Representative: **Hoffmann Eitle**
**Patent- und Rechtsanwälte PartmbB**
**Arabellastraße 30**
**81925 München (DE)**

(56) References cited:
**CN-A- 106 995 429**
**CN-A- 112 047 920**
**CN-B- 102 875 522**
**KR-A- 20080 018 650**
**KR-A- 20140 053 129**
**KR-B1- 101 809 663**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 273 134 B1

## Description

### [TECHNICAL FIELD]

Cross-reference to Related Application(s)

This application claims the benefit of Korean Patent Application No. 10-2021-0075600 filed on June 10, 2021 and Korean Patent Application No. 10-2022-0069132 filed on June 7, 2022 with the Korean Intellectual Property Office.

This invention relates to a method for isolating lactide racemate with high yield and high efficiency without side reactions.

### [BACKGROUND ART]

Polylactic acid not only has biodegradability, but also has excellent mechanical properties such as tensile strength and elasticity, and is being widely used in various fields. Although polylactic acid is homopolymer, it may have various structures due to stereoregularity. Polylactic acid is generally prepared through ring opening polymerization of lactide(LT), but since optical isomers of lactide exist, the properties of polylactic acid may vary according to the arrangement of such optical isomers in the repeat unit.

Meanwhile, lactide is lactic acid dimer, which is cyclic di-ester of lactic acid(2-hydroxypropionic acid, LA), and it was prepared by a reactive distillation method wherein lactic acid is subjected to a dehydration reaction to obtain lactic acid oligomer having relatively low molecular weight as an intermediate, and it is heated to 180°C or more in the presence of a catalyst and decompressed to depolymerize-cyclize, thus forming lactide, and then, it is discharged as steam outside a reactor

Since lactic acid used for the preparation of lactide is a chiral molecule having D-(or (R)-) and L-(or (S)-) enantiomers, lactide produced from the lactic acid exists as three optical isomers having two stereocenters, specifically, L-lactide(L-LT) or (S,S)-lactide formed from two molecules of L-lactic acid, D-lactide(D-LT) or (R,R)-lactide formed from two molecules of D-lactic acid, and meso-lactide(meso-LT) or (R,S)-lactide formed from each one molecule of L-lactic acid and D-lactic acid. Besides the optical isomer mixture, in case lactide is prepared by the reactive distillation method, unreacted lactic acid and lactic acid derivatives may also exist as impurities.

Although a lactide mixture having optical purity of 90% or more, namely, containing 90% or more of L-lactide or D-lactide may be prepared using a selective synthesis method when preparing lactide, it is difficult to produce at high purity of 99% or more. And, since meso-lactide included in the products is rapidly hydrolyzed compared to L-lactide and D-lactide, if the content of meso-lactide in the products is high, hydrolysis is generally promoted. And, lactic acid or lactic acid oligomer increases acidity and inhibits formation of high quality polylactide. Thus, there was a demand for a purification method capable of removing meso-lactide, lactic acid and lactic acid derivatives while containing L-lactide or D-lactide at high purity, according to the purpose of use.

Recently, methods of utilizing meso-lactide produced during the preparation of lactide from lactic acid are being diversely researched and developed.

For example, meso-lactide is converted into L-lactide and D-lactide through racemization. However, lactide converted through racemization is a racemate wherein the rate of L-lactide and D-lactide is 1:1, which are difficult to isolate because of identical physical and chemical properties.

As a method for isolating the racemate, melt-crystallization, simultaneous crystallization using seed introduction, use of chiral solvent, and the like have been suggested. However, the melt-crystallization can be conducted only when the L-lactide rate is 80% or more. And, as a method of using seed introduction and an ethanol solvent, L-lactide is introduced as a seed, and then, L-lactide is isolated using an ethanol solvent, but this method is effective only when the L-lactide rate is 60% or more.

Thus, there is demand for the development of a method capable of isolating lactide racemate with high yield and high efficiency without concern about side reactions, even when the rate of L-lactide is low.

CN102875522 discloses a method for purification of L-lactide from rac-lactide using ethylacetate and dichloromethane.

### [DETAILED DESCRIPTION OF THE INVENTION]

### [TECHNICAL PROBLEM]

It is an object of the invention to provide a method capable of isolating lactide racemate with high yield and high efficiency, without concern about side reactions, even when the rate of L-lactide is low.

**[TECHNICAL SOLUTION]**

In order to achieve the object, there is provided a method for isolating lactide racemate comprising:

introducing L-lactide into lactide racemate and mixing them to prepare a mixture (step 1); and
introducing an extraction solvent into the mixture, to selectively extract L-lactide from the lactide racemate (step 2), wherein the extraction solvent comprises an amine-based compound or an amide-based compound.

Hereinafter, the invention will be explained in detail according to each step.

**(Step 1)**

In the isolation method of lactide racemate of the invention, step 1 is a step of introducing L-lactide into lactide racemate and mixing them to prepare a mixture.
The lactide racemate is a mixture of optical isomers in which L-lactide and D-lactide are mixed in an equal amount, and specifically, it comprises L-lactide and D-lactide at a weight ratio of 1:1.
The lactide racemate may be prepared through racemization of meso-lactide produced from side reactions during the preparation of lactide using lactic acid. Thus, the invention may further comprise a step of conducting racemization of meso-lactide to prepare lactide racemate comprising L-lactide and D-lactide, before step 1 of introducing L-lactide into lactide racemate and mixing them.
The L-lactide introduced in lactide racemate serves as a crystal seed, during the extraction reaction for isolating lactide racemate.
The L-lactide may be introduced in an amount of 10 to 70 parts by weight, based on 100 parts by weight of the lactide racemate. More specifically, the L-lactide may be introduced in such an amount that the content of L-lactide may become greater than 50 wt% and 80 wt% or less, more specifically, greater than 50 wt%, or 52 wt% or more, or 55 wt% or more, and 80 wt% or less, or 75 wt% or less, or 70 wt% or less, or 65 wt% or less, or 60 wt% or less, or less than 60 wt%, based on the total weight of the mixture obtained after introducing L-lactide in lactide racemate.
As the introduction amount of L-lactide and the content of L-lactide in the mixture are higher, L-lactide may be obtained with higher purity. Thus, in order to improve extraction selectivity and improve the decrease of impurity content, the L-lactide may be introduced in such an amount that the content of L-lactide may become 65 wt% or more, or 70 wt% or more, and 80 wt% or less, or 75 wt% or less, based on the total weight of the mixture obtained after introducing L-lactide in lactide racemate. However, in the present disclosure, since an optimal extraction solvent is used in the subsequent extraction process, even if the content of L-lactide in the mixture is as low as 60 wt% or less, or less than 60 wt%, it is possible to isolate L-lactide. Thus, the L-lactide may be introduced in such an amount that the content of L-lactide may become greater than 50 wt%, or 52wt% or more, or 55 wt% or more, and 60 wt% or less, or less than 60 wt%, or 58 wt% or less, based on the total weight of the mixture.

**(Step 2)**

In the isolation method of lactide racemate of the invention, step 2 is a step of introducing an extraction solvent into the mixture prepared in step 1, to selectively extract L-lactide from the lactide racemate,
**[0022]** As the extraction solvent, an amine-based compound, or an amide-based compound is used. These extraction solvents are non-chiral compounds without chiral properties, have polarities, and may exhibit excellent extraction results due to shift of eutectic point of three-component system phase of L-lactide, D-lactide, and an extraction solvent. Thus, these extraction solvents exhibit excellent extraction efficiency compared to alcohol, and the like conventionally used during the isolation of lactide racemate, and particularly, amine-based compounds such as TEA, and the like may exhibit high extraction efficiency even when the content of L-lactide in the mixture is low.
And, the extraction solvent exists as liquid at room temperature($20\pm5$°C), and it may be a polar solvent having a melting point of 25°C or less and a boiling point of 80°C or more. More specifically, the extraction solvent has a melting point of 25°C or less, or 10°C or less, and -150°C or more, or -120°C or more, and a boiling point of 80°C or more or 100°C or more, and 300°C or less, or 250°C or less. When the above melting point and boiling point conditions are met, stable and excellent extraction efficiency may be exhibited while minimizing generation of side reactions.
The amine-based compound is a tertiary amine represented by the following Chemical Formula 1:

[Chemical Formula 1]

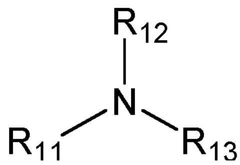

In the Chemical Formula 1,
$R_{11}$ to $R_{13}$ are each independently a $C_{1-20}$ linear or branched alkyl.

As specific examples of the amine-based compound, trimethylamine, triethylamine, tri-n-propylamine, tri-n-butylamine, tri-n-octylamine, tridodecylamine, trioctadecylamine, triisopropylamine, triisobutylamine, tri-t-butylamine, tris(2-ethylhexyl)amine, N,N-dilmethyloctylamine, N,N-dimethyldodecylamine, N,N-dimethyl-n-octadecylamine, or N,N-diethyldodecylamine, and the like may be mentioned, and one of them or mixtures of two or more of them may be used.

Among them, considering remarkable isolation effect, the amine-based compound may be a tertiary amine of the Chemical Formula 1 wherein $R_{11}$ to $R_{13}$ are each independently a $C_{1-9}$ linear alkyl. More specifically, the amine-based compound may be triethylamine, or tri-n-octylamine.

The amide-based compound is a compound represented by the following Chemical Formula 2:

[Chemical Formula 2]

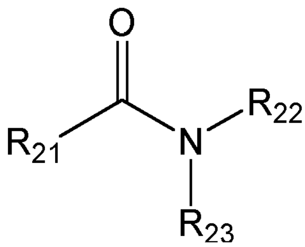

In the Chemical Formula 2,
each of $R_{21}$ to $R_{23}$ is hydrogen, or is independently a $C_{1-20}$ linear or branched alkyl.

The amide-based compounds represented by the Chemical Formula 2 may exhibit more excellent isolation effect, compared to dimethylformamide, and the like that do not satisfy the $R_{21}$ to $R_{23}$ substituent requirements.

As specific examples of the amide-based compounds, formamide, or dimethylacetamide, and the like may be mentioned, and one of them or mixtures of two or more of them may be used.

The extraction solvent may be used in an amount of 1 to 10 parts by weight, based on 1 part by weight of L-lactide existing in the mixture. If the amount of the extraction solvent used is too small, extraction efficiency may not be sufficient, and if the amount of the extraction solvent used is too large, a process for removing the extraction solvent should be conducted later, thus deteriorating processability. When the extraction solvent is used within the above range, lactide racemate may be isolated with excellent extraction efficiency without additional process for removing extraction solvents. More specifically, the extraction solvent may be used in an amount of 1 part by weight or more, or 2 parts by weight or more, or 2.5 parts by weight or more, or 3 parts by weight or more, or 3.1 parts by weight or more, or 3.2 parts by weight or more, and 10 parts by weight or less, or 8 parts by weight or less, or 6.5 parts by weight or less, or 5 parts by weight or less, or 4 parts by weight or less, or 3.6 parts by weight or less, or 3.5 parts by weight or less, based on 1 part by weight of L-lactide existing in the mixture.

And, after introducing the extraction solvent in the mixture, a mixing process for uniform mixing and the resulting increase in isolation efficiency may be optionally further conducted.

Wherein, the mixing process may be conducted by a common method such as a stirrer, a mixer, and the like, and a mixing speed, time, and the like may be appropriately selected.

And, the step 2 may be conducted at a temperature of 10 to 40°C. Specifically, it may be conducted at a temperature of 10°C or more, or 15°C or more, and 40°C or less, or 30°Cor less, or 25°C or less, and more specifically, at a room temperature of 20±5°C. When the step 2 is conducted within the above temperature range, sufficiency extraction efficiency may be realized without concern about generation of side reactions.

As the result of the reaction, L-lactide is isolated from lactide racemate.

Specifically, after introducing the extraction solvent, and then, completing the optional mixing process, precipitated solids are separated and removed from the obtained reaction product through filtration, thus obtaining a filtrate.

The precipitated solids are D-lactide not dissolved in the extraction solvent and the remainder of L-lactide. An

isolation process of them may be conducted by a common method, and specifically, may be isolated through filtration.

Meanwhile, in gas chromatography analysis of the filtrate, L-lactide was included in high content, specifically, in the content of 90 wt% or more, or 92 wt% or more, or 93 wt% or more, or 94 wt% or more, based on the total weight of the filtrate. And, the content of by-products formed by side reactions such as hydrolysis was less than 1 wt%, or 0.95 wt% or less, based on the total weight of the filtrate.

**[ADVANTAGEOUS EFFECTS]**

As explained above, the isolation method of lactide racemate according to the invention may isolate lactide racemate with high yield and high efficiency, without concern about side reactions, even when the rate of L-lactide is low. Thus, lactide of high purity isolated by the method may be usefully used for the production of polylactide having high molecular weight.

**[BRIEF DESCRIPTION OF THE DRAWINGS]**

Fig. 1 is a graph showing the results of evaluating L-LT extraction selectivity according to introduced seed and L-LT rate.
Fig. 2 is a graph showing the results of gas chromatography analysis of Example 3.
Fig. 3 is a graph showing the result of gas chromatography analysis of Example 6.
Fig. 4 is a graph showing the result of gas chromatography analysis of Comparative Example 3.

**[DETAILED DESCRIPTION OF THE EMBODIMENTS]**

Hereinafter, embodiments of the invention will be explained in more detail in the following examples. However, these examples are presented only as the illustrations of the invention, and scope of the invention is not limited thereby.

**Example 1**

Into 0.25g of Rac-LT(weight ratio of L-LT:D-LT=1:1), 0.16g of L-LT (introduction amount of L-LT, based on 100 parts by weight of Rac-LT = 64 parts by weight) was introduced and mixed, thus preparing a mixture wherein the weight ratio of L-LT:D-LT is 7:3 (L-LT content based on the total weight of the mixture = 70 wt%).

Into the mixture, 1.30ml of triethylamine (TEA, melting point: -114.70°C, boiling point: 89.3°C) (corresponding to 3.28 parts by weight, based on 1 part by weight of L-lactide existing in the mixture) was introduced, and stirred at room temperature for 1 hour. The resulting solution was in the state of an emulsion, and the solid parts were separated and removed by filtering through a filter, thus obtaining a filtrate.

**Example 2**

The same procedure as Example 1 was conducted, except that 0.07g of L-LT (introduction amount of L-LT, based on 100 parts by weight of Rac-LT = 28 parts by weight) was introduced into 0.25g of Rac-LT(weight ratio of L-LT:D-LT = 1:1), and mixed such that the weight ratio of L-LT:D-LT became 6:4 (L-LT content based on the total weight of the mixture = 60 wt%).

**Example 3**

The same procedure as Example 1 was conducted, except that 0.03g of L-LT (introduction amount of L-LT, based on 100 parts by weight of Rac-LT = 12 parts by weight) was introduced into 0.25g of Rac-LT(weight ratio of L-LT:D-LT = 1:1), and mixed such that the weight ratio of L-LT:D-LT became 5.5:4.5 (L-LT content based on the total weight of the mixture = 55 wt%).

**Example 4**

The same procedure as Example 1 was conducted, except that 0.84 ml of formamide (FA, melting point: 2°C, boiling point: 210°C) (corresponding to 3.31 parts by weight, based on 1 part by weight of L-lactide existing in the mixture) was introduced instead of 1.30ml of triethylamine.

**Example 5**

The same procedure as Example 2 was conducted, except that 0.84 ml of formamide (corresponding to 4.95 parts by weight, based on 1 part by weight of L-lactide existing in the mixture) was introduced instead of 1.30ml of triethylamine.

**Example 6**

The same procedure as Example 3 was conducted, except that 0.84 ml of formamide (corresponding to 6.18 parts by weight, based on 1 part by weight of L-lactide existing in the mixture) was introduced instead of 1.30ml of triethylamine.

**Example 7**

The same procedure as Example 1 was conducted, except that 1.17 ml of tri-n-octylamine (melting point: -34.0°C, boiling point: 367°C) (corresponding to 3.31 parts by weight, based on 1 part by weight of L-lactide existing in the mixture) was introduced instead of 1.30ml of triethylamine.

**Example 8**

The same procedure as Example 2 was conducted, except that 1.17 ml of tri-n-octylamine (corresponding to 4.87 parts by weight, based on 1 part by weight of L-lactide existing in the mixture) was introduced instead of 1.30ml of triethylamine.

**Example 9**

The same procedure as Example 1 was conducted, except that 1.17 ml of tri-n-octylamine (corresponding to 6.12 parts by weight, based on 1 part by weight of L-lactide existing in the mixture) was introduced instead of 1.30ml of triethylamine.

**Comparative Example 1**

The same procedure as Example 1 was conducted, except that 1.204 ml of ethanol (EtOH, melting point: -114.1°C, boiling point: 78.4°C) was introduced instead of 1.30ml of triethylamine.

**Comparative Example 2**

The same procedure as Example 2 was conducted, except that 1.204 ml of ethanol was introduced instead of 1.30ml of triethylamine.

**Comparative Example 3**

The same procedure as Example 3 was conducted, except that 1.204 ml of ethanol was introduced instead of 1.30ml of triethylamine.

**Comparative Example 4**

The same procedure as Example 1 was conducted, except that 0.92ml of 1,4-dioxane (melting point: 11°C, boiling point: 101°C) was introduced instead of 1.30ml of triethylamine.

**Comparative Example 5**

The same procedure as Example 2 was conducted, except that 0.92ml of 1,4-dioxane was introduced instead of 1.30ml of triethylamine.

**Comparative Example 6**

The same procedure as Example 3 was conducted, except that 0.92ml of 1,4-dioxane was introduced instead of

1.30ml of triethylamine.

**Comparative Example 7**

The same procedure as Example 1 was conducted, except that L-LT was not introduced into Rac-LT as a seed.
Specifically, into Rac-LT(weight ratio of L-LT:D-LT = 1:1), 1.30ml of triethylamine(corresponding to 7.6 parts by weight, based on 1 part by weight of L-lactide existing in the mixture) was introduced, and stirred at room temperature for 1 hour.
However, in the resulting solution, separation of solid corresponding to D-LT did not occur.

**Experimental Example**

(1) L-LT extraction selectivity

For each filtrate obtained in Examples and Comparative Examples, the weight ratio of L-LT:D-LT was measured, and from the results, L-LT extraction selectivity was evaluated.
Specifically, about 60$\mu\ell$ of each filtrate obtained in Example and Comparative Example was taken and diluted in 1ml of acetonitrile(MeCN) to prepare a sample for gas chromatography(GC) analysis. For the prepared sample, GC analysis was conducted under the following conditions. In the chromatogram obtained as the result of GC analysis, the total peak area and L-LT peak area were respectively calculated, and L-LT extraction selectivity(%) was calculated according to the following Mathematical Formula 1.

[Mathematical Formula 1]

$$\text{L-LT extraction selectivity(\%)} = \text{(L-LT peak area/total peak area)} \times 100$$

In the Mathematical Formula 1, the total peak area and L-LT peak area were respectively calculated through integral of each peak.

<GC analysis conditions>

GC/FID, Shimadzu
Column: CP-Chirasil
Dilution solvent: MeCN(acetonitrile)
Injection volume: 1$\mu\ell$
Injection port temperature: 200°C
Carrier gas: $N_2$
FID (flame photometric detector) Temperature: 275°C
Column temperature:

Carrier flow rate (m$\ell$/min ): 1.5 ml/min
Analysis time: 19.60 min
The results were shown in the following Table 1, and Figs. 1 to 4.

Fig. 1 is a graph showing the results of evaluating L-LT extraction selectivity according to introduced seed and L-LT rate, and Figs. 2 to 4 are graphs respectively showing the gas chromatography analysis results of Example 3, Example 6 and Comparative Example 3.
In the gas chromatography analysis graphs of Figs. 2 to 4, although x axis starts at O min where the analysis began, it is enlarged from 8 min where peaks began to be observed so as to clearly identify the results.

(2) Impurity content(%)

From the GC analysis results, the contents of impurities generated during the isolation of racemate lactides according to Examples and Comparative Examples were measured.
Specifically, in the GC analysis chromatogram, the areas of peaks corresponding to impurities such as meso-LT

peak and unknown peaks (hereinafter, simply referred to as 'impurity peaks') were respectively calculated, and impurity content(%) was calculated according to the following Mathematical Formula 2.

[Mathematical Formula 2]

$$\text{Impurity content(\%)} = (\text{total area of impurity peaks} / \text{total peak area}) \times 100$$

In the Mathematical Formula 2, the total peak area and the area of impurity peak are respectively calculated through integral of each peak, and the total area of impurity peaks means the sum of impurity peak areas calculated through integral.

[Table 1]

| | Weight ratio of L-LT:D-LT in the mixture, after introducing L-LT | Extraction solvent | Weight ratio of L-LT:D-LT in the finally obtained filtrate | L-LT extraction selectivity (%) | Impurity content (%) |
|---|---|---|---|---|---|
| Example 1 | 70:30 | TEA | 94.5:5.5 | 94.491 | 0 |
| Example 2 | 60:40 | TEA | 93.5:6.5 | 93.538 | 0 |
| Example 3 | 55:45 | TEA | 93.9:6.1 | 93.926 | 0 |
| Example 4 | 70:30 | FA | 92.0:8.0 | 92.045 | 0.643 |
| Example 5 | 60:40 | FA | 84.0:16.0 | 84 | 0.884 |
| Example 6 | 55:45 | FA | 66.1:33.9 | 66.069 | 0.928 |
| Example 7 | 70:30 | Tri-n-octylamine | 93.5:6.5 | 93.529 | 0 |
| Example 8 | 60:40 | Tri-n-octylamine | 92.9:7.1 | 92.929 | 0 |
| Example 9 | 55:45 | Tri-n-octylamine | 93.2:6.8 | 92.884 | 0 |
| Comparative Example 1 | 70:30 | EtOH | 94.0:6.0 | 93.963 | 11.782 |
| Comparative Example 2 | 60:40 | EtOH | 94.3:5.7 | 94.266 | 6.940 |
| Comparative Example 3 | 55:45 | EtOH | 86.5:13.5 | 86.547 | 5.227 |
| Comparative Example 4 | 70:30 | 1,4-dioxane | 79.2:20.8 | 79.252 | 0 |
| Comparative Example 5 | 60:40 | 1,4-dioxane | 66.3:33.7 | 66.274 | 0 |
| Comparative Example 6 | 55:45 | 1,4-dioxane | 57.9:42.1 | 57.898 | 0 |

As the results of experiment, Examples 1-9 wherein TEA, FA or Tri-n-octylamine was used as an extraction solvent exhibited excellent L-LT extraction selectivities and remarkably decreased impurity contents.

Specifically, in case a L-LT rate in the mixture after introducing L-LT was 60 wt% or more, Examples, 1, 2, 4, 5, 7 and 8 wherein TEA, FA or Tri-n-octylamine was used as an extraction solvent exhibited L-LT extraction selectivities equivalent to or more excellent than those of Comparative Examples 1, 2, 4 and 5.

And, in case a L-LT rate in the mixture after introducing L-LT was less than 60%, specifically as low as 55%, the conventional extraction solvent EtOH exhibited decreased L-LT extraction selectivity of 86.547%, while TEA and Tri-n-octylamine still exhibited high L-LT extraction selectivities of 92.045% and 92.884%, respectively.

Meanwhile, in the case of Example 6 wherein FA was used as an extraction solvent, L-LT extraction selectivity was 66.069%, which was lower than that of Comparative Example 3 using EtOH. However, the content of impurities generated during the isolation of lactide racemate using FA was 0.928%, while the content of impurities generated during the isolation of lactide racemate using EtOH was 5.227%, which was remarkably high. Thus, it can be seen that in case a L-LT content is low, when FA is used as an extraction solvent, L-LT extraction selectivity is rather lower compared to the case of using EtOH, but since impurity content may be remarkably decreased, L-LT can be obtained with higher purity.

And, as shown in the GC analysis graphs of Figs. 2 to 4, in the case of Example 3 using TEA, peaks other than L-LT and D-LT did not exist in the GC analysis graph. To the contrary, in the case of Comparative Example 3 using an EtOH solvent, unknown peaks other than L-LT and D-LT were observed in the GC analysis graph, confirming the possibility of side reactions of lactide such as hydrolysis, and the like.

And, in the case of Example 6 using FA, very small meso-LT peak was observed in the section of retention time-10 minutes or more in the GC analysis graph, thus confirming the existence of side reactions. However, since the rate is very low, the content of by-products was also very small.

## Claims

1. A method for separating lactide racemate comprising:

   introducing L-lactide into lactide racemate and mixing them to prepare a mixture; and
   introducing an extraction solvent into the mixture, to selectively extract L-lactide from the lactide racemate,
   wherein the extraction solvent comprises an amine-based compound or an amide-based compound,
   the amine-based compound is a tertiary amine represented by the following Chemical Formula 1, and
   the amide-based compound is a compound represented by the following Chemical Formula 2:

   [Chemical Formula 1]

   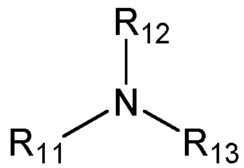

   in the Chemical Formula 1,
   $R_{11}$ to $R_{13}$ are each independently a $C_{1-20}$ linear or branched alkyl,

   [Chemical Formula 2]

   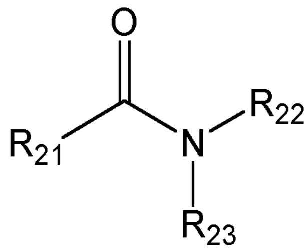

   in the Chemical Formula 2,
   each of $R_{21}$ to $R_{23}$ is hydrogen, or is independently a $C_{1-20}$ linear or branched alkyl.

2. The method according to claim 1, wherein the extraction solvent is liquid, and has a melting point of 25°C or less and a boiling point of 80°C or more.

3. The method according to claim 1, wherein the extraction solvent is a non-chiral compound.

4. The method according to claim 1, wherein the amine-based compound is trimethylamine, triethylamine, tri-n-propylamine, tri-n-butylamine, tri-n-octylamine, tridodecylamine, trioctadecylamine, triisopropylamine, triisobutylamine, tri-t-butylamine, tris(2-ethylhexyl)amine, N,N-dimethyloctylamine, N,N-dimethyldodecylamine, N,N-dimethyl-n-octadecylamine, or N,N-diethyldodecylamine.

5. The method according to claim 1, wherein the amine-based compound is triethylamine, or tri-n-octylamine.

6. The method according to claim 1, wherein the amide-based compound is formamide, or dimethylacetamide.

7. The method according to claim 1, wherein the extraction solvent is used in an amount of 1 to 10 parts by weight, based on 1 part by weight of L-lactide existing in the mixture.

8. The method according to claim 1, wherein the L-lactide is introduced in an amount of 10 to 70 parts by weight, based on

100 parts by weight of the lactide racemate.

9. The method according to claim 1, wherein the L-lactide is introduced in such an amount that the content of L-lactide in the mixture becomes greater than 50 wt% and 80 wt% or less, based on the total weight of the mixture.

10. The method according to claim 1, wherein the L-lactide is introduced in such an amount that the content of L-lactide in the mixture becomes greater than 50 wt% and less than 60 wt%, based on the total weight of the mixture.

## Patentansprüche

1. Verfahren zur Trennung eines Lactid-Racemats, umfassend:

das Einbringen von L-Lactid in Lactid-Racemat und das Mischen, so dass ein Gemisch hergestellt wird; und
das Einbringen eines Extraktionslösungsmittels in das Gemisch zur selektiven Extraktion von L-Lactid aus dem Lactid-Racemat,
worin das Extraktionslösungsmittel eine Amin-basierte Verbindung oder eine Amid-basierte Verbindung umfasst,
die Amin-basierte Verbindung ein durch die folgende chemische Formel 1 dargestelltes tertiäres Amin ist und die Amid-basierte Verbindung eine durch die folgende chemische Formel 2 dargestellte Verbindung ist:

[chemische Formel 1]

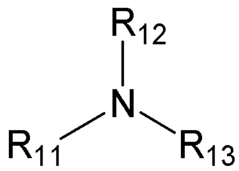

wobei, in der chemischen Formel 1,
$R_{11}$ bis $R_{13}$ jeweils unabhängig ein lineares oder verzweigtes $C_{1-20}$-Alkyl ist,

[chemische Formel 2]

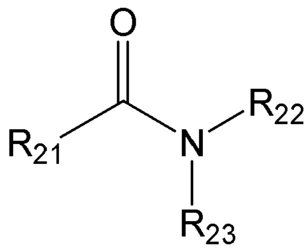

wobei, in der chemischen Formel 2,
jedes von $R_{21}$ bis $R_{23}$ Wasserstoff ist oder unabhängig ein lineares oder verzweigtes $C_{1-20}$-Alkyl ist.

2. Verfahren gemäß Anspruch 1, worin das Extraktionslösungsmittel flüssig ist und einen Schmelzpunkt von 25 °C oder weniger und einen Siedepunkt von 80 °C oder mehr aufweist.

3. Verfahren gemäß Anspruch 1, worin das Extraktionslösungsmittel eine nicht-chirale Verbindung ist.

4. Verfahren gemäß Anspruch 1, worin die Amin-basierte Verbindung Trimethylamin, Triethylamin, Tri-n-propylamin, Tri-n-butylamin, Tri-n-octylamin, Tridodecylamin, Trioctadecylamin, Triisopropylamin, Triisobutylamin, Tri-t-butylamin, Tris(2-ethylhexyl)amin, N,N-Dimethyloctylamin, N,N-Dimethyldodecylamin, N,N-Dimethyl-n-octadecylamin oder N,N-Diethyldodecylamin ist.

5. Verfahren gemäß Anspruch 1, worin die Amin-basierte Verbindung Triethylamin oder Tri-n-octylamin ist.

**6.** Verfahren gemäß Anspruch 1, worin die Amid-basierte Verbindung Formamid oder Dimethylacetamid ist.

**7.** Verfahren gemäß Anspruch 1, worin das Extraktionslösungsmittel in einer Menge von 1 bis 10 Gewichtsteilen, basierend auf 1 Gewichtsteil von im Gemisch vorhandenen L-Lactid, verwendet wird.

**8.** Verfahren gemäß Anspruch 1, worin das L-Lactid in einer Menge von 10 bis 70 Gewichtsteilen, basierend auf 100 Gewichtsteilen des Lactid-Racemats, eingeführt wird.

**9.** Verfahren gemäß Anspruch 1, worin das L-Lactid in einer solchen Menge zugegeben wird, dass der Gehalt an L-Lactid in der Mischung, basierend auf dem Gesamtgewicht des Gemischs, mehr als 50 Gew.-% und 80 Gew.-% oder weniger wird.

**10.** Verfahren gemäß Anspruch 1, worin das L-Lactid in einer solchen Menge zugegeben wird, dass der Gehalt an L-Lactid in der Mischung, basierend auf dem Gesamtgewicht des Gemischs, mehr als 50 Gew.-% und weniger als 60 Gew.-% wird.

**Revendications**

**1.** Procédé de séparation d'un mélange racémique de lactide comprenant :

une introduction de L-lactide dans le mélange racémique de lactide et le fait de les mélanger pour préparer un mélange ;
et
une introduction d'un solvant d'extraction dans le mélange, pour extraire sélectivement le L-lactide du mélange racémique de lactide,
dans lequel le solvant d'extraction comprend un composé à base d'amine ou un composé à base d'amide,
le composé à base d'amine est une amine tertiaire représentée par la formule chimique 1 suivante, et
le composé à base d'amide est un composé représenté par la formule chimique 2 suivante :

[Formule Chimique 1]

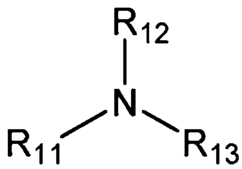

dans la formule chimique 1,
$R_{11}$ à $R_{13}$ sont chacun indépendamment un alkyle linéaire ou ramifié en $C_{1-20}$,

[Formule Chimique 2]

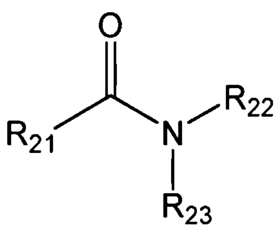

dans la formule chimique 2,
chacun de $R_{21}$ à $R_{23}$ est un hydrogène, ou est indépendamment un alkyle linéaire ou ramifié en $C_{1-20}$.

**2.** Procédé selon la revendication 1, dans lequel le solvant d'extraction est liquide, et présente un point de fusion de 25 °C ou inférieur et un point d'ébullition de 80 °C ou plus.

3. Procédé selon la revendication 1, dans lequel le solvant d'extraction est un composé non chiral.

4. Procédé selon la revendication 1, dans lequel le composé à base d'amine est le triméthylamine, le triéthylamine, le tri-n-propylamine, le tri-n-butylamine, le tri-n-octylamine, le tridodécylamine, le trioctadécylamine, le triisopropylamine, le triisobutylamine, le tri-t-butylamine, le tris(2-éthylhexyl)amine, le N,N-diméthyloctylamine, le N,N-diméthyldodécylamine, le N,N-diméthyl-n-octadécylamine, ou le N,N-diéthyldodécylamine.

5. Procédé selon la revendication 1, dans lequel le composé à base d'amine est le triéthylamine, ou le tri-n-octylamine.

6. Procédé selon la revendication 1, dans lequel le composé à base d'amide est le formamide, ou le diméthylacétamide.

7. Procédé selon la revendication 1, dans lequel le solvant d'extraction est utilisé dans une quantité de 1 à 10 parties en poids, sur la base de 1 partie en poids de L-lactide présente dans le mélange.

8. Procédé selon la revendication 1, dans lequel le L-lactide est introduit dans une quantité de 10 à 70 parties en poids, sur la base de 100 parties en poids du mélange racémique de lactide.

9. Procédé selon la revendication 1, dans lequel le L-lactide est introduit en une quantité telle que la teneur en L-lactide dans le mélange devienne supérieure à 50 % en poids et inférieure ou égale à 80 % en poids, sur la base du poids total du mélange.

10. Procédé selon la revendication 1, dans lequel le L-lactide est introduit en une quantité telle que la teneur en L-lactide dans le mélange devienne supérieure à 50 % en poids et inférieure à 60 % en poids, sur la base du poids total du mélange.

【Fig. 1】

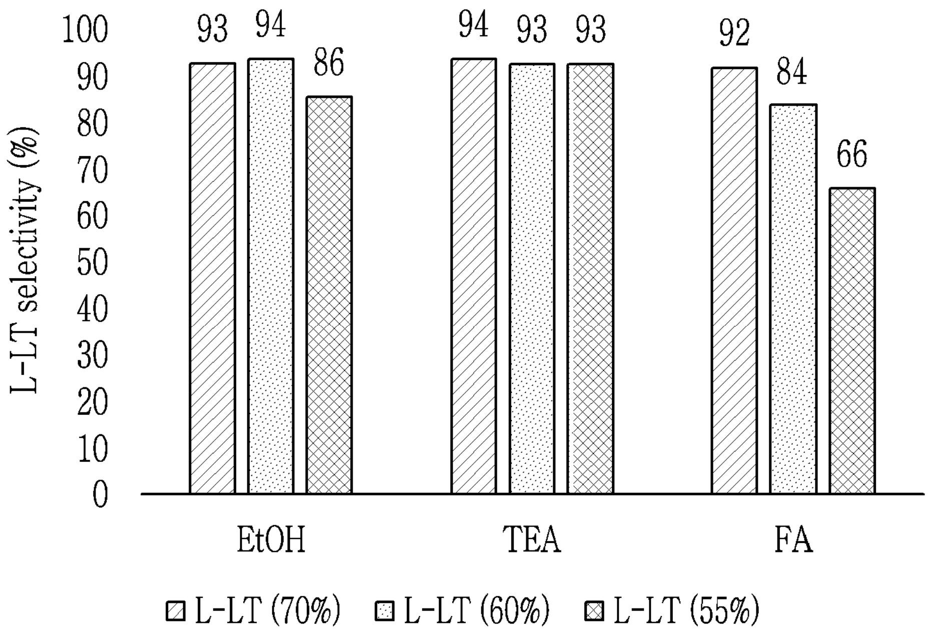
L-LT selectivity (%)
100
90
80
70
60
50
40
30
20
10
0
93
94
86
94
93
93
92
84
66
EtOH
TEA
FA
L-LT (70%)
L-LT (60%)
L-LT (55%)

【Fig. 2】

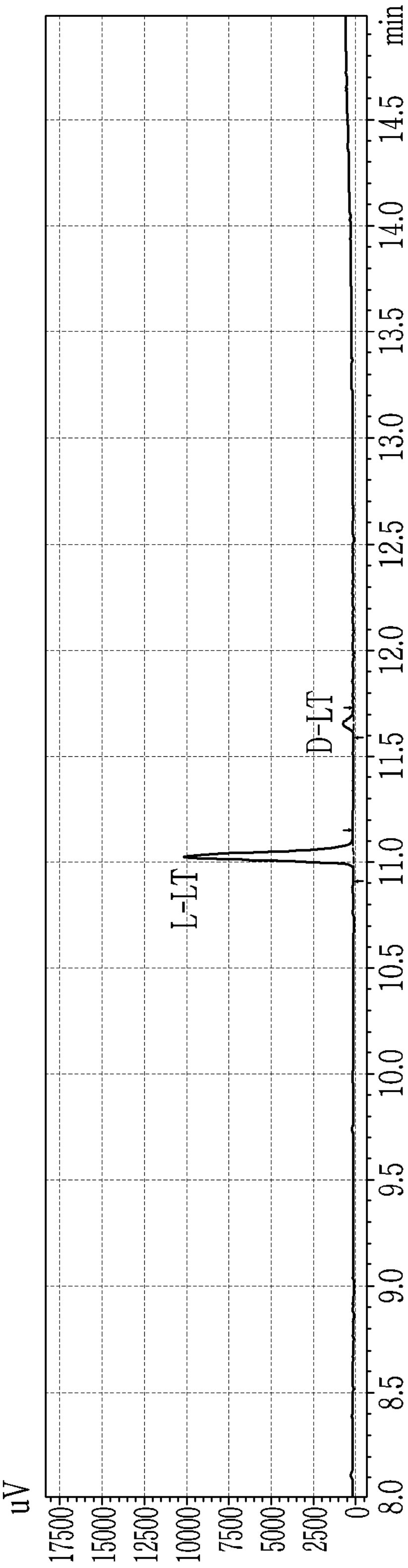
uV
17500
15000
12500
10000
7500
5000
2500
0
8.0
8.5
9.0
9.5
10.0
10.5
11.0
11.5
12.0
12.5
13.0
13.5
14.0
14.5
min
L-LT
D-LT

【Fig. 3】

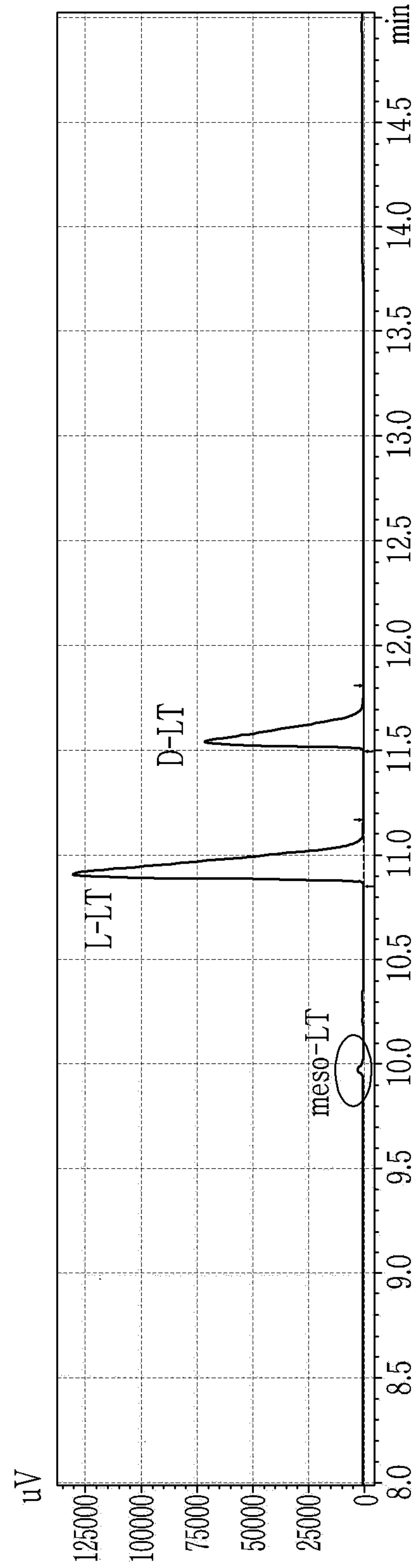
uV
125000
100000
75000
50000
25000
0
8.0
8.5
9.0
9.5
10.0
10.5
11.0
11.5
12.0
12.5
13.0
13.5
14.0
14.5
min
L-LT
D-LT
meso-LT

【Fig. 4】

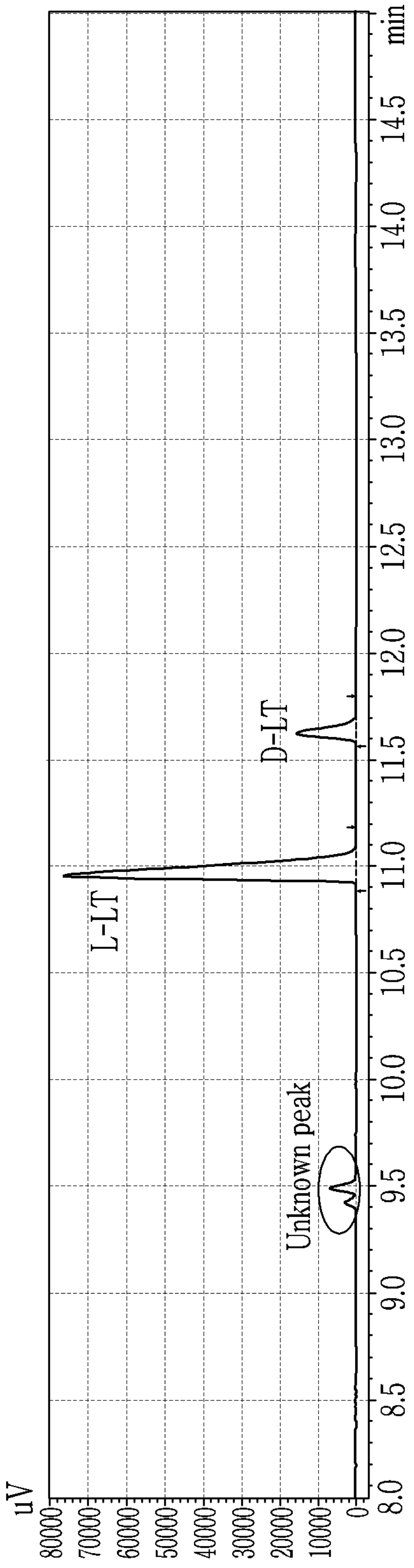
uV
80000
70000
60000
50000
40000
30000
20000
10000
0
8.0
8.5
9.0
9.5
10.0
10.5
11.0
11.5
12.0
12.5
13.0
13.5
14.0
14.5
min
Unknown peak
L-LT
D-LT

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020210075600
- KR 1020220069132
- CN 102875522